# EUROPEAN PATENT APPLICATION

(11) **EP 3 699 273 A1**
(43) Date of publication of application: **26.08.2020**
(21) Application number: 19158656.9
(22) Date of filing: 21.02.2019
(51) Int. Cl.: C12N 9/18

(54) **LC-CUTINASE**

(71) Applicant: EVOXX Technologies GmbH, 40789 Monheim (DE)
(72) Inventor: LEGGEWIE, Christian, Dr., 45479 Mülheim (DE); FERNANDEZ, Layla, Dr., 50858 Köln (DE); TUROWSKI, Stephanie, 42799 Leichlingen (DE); GISIN, Jonathan, Dr., 40789 Monheim (DE); PULS, Michael, Dr., 40789 Monheim (DE)
(74) Representative: Michalski Hüttermann & Partner Patentanwälte mbB

(57) **Abstract**

The invention relates to enzyme technology, in particular to enzymes having esterase activity, which e.g. are used in treatment of waste water or composite materials. In another aspect, the invention relates to a polyester compositions treatment agent, in particular PET compositions treatment agent, which comprise a cutinase as defined herein. Further, the invention relates to a method for treatment of a polyester composition, in particular a PET composition, by use of such an agent. Furthermore, the invention relates to the use of a cutinase as defined herein in waste water, sewage, or composite material solution treatment.

## Description

The invention relates to enzyme technology, in particular to enzymes having esterase activity, which e.g. are used in treatment of waste water or composite materials. In another aspect, the invention relates to a polyester compositions treatment agent, in particular PET compositions treatment agent, which comprise a cutinase as defined herein. Further, the invention relates to a method for treatment of a polyester composition, in particular a PET composition, by use of such an agent. Furthermore, the invention relates to the use of a cutinase as defined herein in waste water, sewage, or composite material solution treatment.

Polyesters, in particular polyethylene-terephthalates (PET), are widely used in several applications, in particular due to their favorable processing conditions and due to their chemical and physical stability. However, PET may pose challenges in recycling and waste management because of said stability. While the importance of plastic recycling has risen over the past decades there is still the need for improved methods for treatment of polyester containing compositions. That is, for the treatment in recycling of composite materials comprising polyester together with other materials such as metal foils or cartonnage suitable methods for separating the different materials are needed. Furthermore, for the treatment of waste water, in particular containing micro-plastics, methods for degradation of the residual plastics are needed, in order to reduce further contamination.

PET Esterases degrade polymeric Polyethyleneglycolterephtalate (PET) into its oligomers, dimers and monomers or even into terephatalic acid (TPA) and ethylene glycol (EG). Several PET-degrading enzymes have been found which are hydrolases, mostly with alpha-beta-hydrolase-typ fold. Among those are enzyme with known functions like e.g. Lipases, Esterases, Cutinases and others. Such enzymes, due to their PET degrading ability are also named PETases. The enzymes are quite stable by nature and hence are suitable for their application in technical processes. In particular, the cutinase originates from a fungus that makes use of the enzymes in their natural function to degrade the cutin layer of leaves in order to reach for the leave-structures underneath. Hence the enzymes have undergone a process of natural evolution that enabled them to stay functional under outside weather conditions. The enzyme is a protein and hence a biological material that is fully biodegradable.

Recently it was found that a cutinase identified by a metagenomic approach in leaf compost (Sulaiman et al. (2011) "Isolation of a Novel Cutinase Homolog with polyester-Degrading Activity from Leaf-Branch Compost by Using a Metagenomic Approach", Appl. Environ. Microbiol. 78(5):1556-1562), shows fast polyester (PET) degradation at 50°C.

Surprisingly, the inventors of the present invention have now found that certain variants of the cutinase described above have a significantly improved performance compared to the wild type cutinase.

It was found that these cutinases provide improved degradation of polyesters, in particular PET. Furthermore, the cutinases according to the invention allow for the removal of micro-polyethylene in waste water by degradation of the polymers into further degradable oligomers and monomers. In addition, the cutinases according to the invention allow for the degradation of polyethylene parts, in particular PET parts, of composite materials, allowing the separation of these materials from the polyethylene.

Therefore, in a first aspect, the present invention is directed to an agent for treatment of polyester compositions, in particular PET compositions, characterized in that it contains a cutinase which has at least 65% sequence identity with the amino acid sequence set forth in SEQ ID NO:1 over the entire length thereof and has, with respect to the numbering according to SEQ ID NO:1, at least one amino acid substitution at at least one of the positions corresponding to the positions 61, 63, 66, 89, 90, 92, 155, 177, 208 and 211, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q.

In a further aspect, the present invention is directed to methods for treatment of a polyester composition, in particular a PET composition, characterized in that in at least one method step an agent according to the invention is added to the polyester composition, wherein the cutinase is reacted with the polyester.

In yet another aspect, the present invention is also directed at the production of a cutinase used in the agent and method according to the invention, wherein the cutinase is expressed by a system selected from the group consisting of *E. coli, Bacillus subtilis, and Bacillus lichniformis.*

These and other aspects, features and advantages of the invention become apparent to the skilled person from the study of the following detailed description and claims. Each feature from one aspect of the invention can be used in any other aspect of the invention. Furthermore, it is clear that the examples provided herein are intended to describe and illustrate the invention but not to restrict it and, in particular, not to limit the invention to these examples.

A cutinase (EC 3.1.1.74), also known as cutin hydrolase, is an enzyme that belongs to the α/β-hydrolases and hydrolyzes cutin. Cutinase is produced by a number of plant pathogenic fungi and bacteria (cutinase enables fungi to break down the ester bond of cutin in the cuticle of plants and thus penetrate the plants).

The observed benefits of the cutinase are manifold. Particularly, the cutinase provides improved degradation of polyesters, in particular PET. Furthermore, the cutinase according to the invention allows for the removal of micro-polyethylene in waste water by degradation of the polymers into further degradable oligomers and monomers. In addition, the cutinases according to the invention allow for the degradation of polyethylene parts, in particular PET parts, of composite materials, allowing the separation of these materials from the polyethylene.

In various embodiments of the invention, the cutinase is a freely available enzyme. This means that the cutinase can act directly with all components of the agent and, if the agent is a liquid, that the cutinase is in direct contact with the solvent of the invention (e.g. water). In further embodiments, the invention may contain cutinase that forms an interaction complex with other molecules or that comprises a "coating". A single or multiple cutinase molecule(s) may be separated from other components of the agent by a surrounding structure. Such a separating structure may be formed by, but is not limited to, vesicles such as a micelle or a liposome. The surrounding structure can also be a virus particle, a bacterial cell or a eukaryotic cell.

The present invention is based on the surprising finding of the inventors that amino acid substitutions at positions of the cutinase described herein improve the overall performance of the agents for treatment of polyester compositions according to the invention.

In various preferred embodiments of the invention, the cutinase used in agents according to the invention is a cutinase having at least 65% sequence identity with the amino acid sequence set forth in SEQ ID NO:1 over the entire length thereof and having, with respect to the numbering according to SEQ ID NO:1, at least one amino acid substitution at at least one of the positions corresponding to the positions 61, 63, 66, 89, 90, 92, 155, 177, 208 and 211, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution T61S at the position corresponding to position 61 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution D63E at the position corresponding to position 63 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution D63T at the position corresponding to position 63 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution S66A at the position corresponding to position 66 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution S66C at the position corresponding to position 66 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution R89K at the position corresponding to position 89 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution R89L at the position corresponding to position 89 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution F90I at the position corresponding to position 90 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution Y92D at the position corresponding to position 92 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution Y92C at the position corresponding to position 92 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution Y92A at the position corresponding to position 92 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution W155A at the position corresponding to position 155 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution W155Y at the position corresponding to position 155 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution V177P at the position corresponding to position 177 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution F208E at the position corresponding to position 208 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution F208Y at the position corresponding to position 208 of SEQ ID NO:1 .

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution N211E at the position corresponding to position 211 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution N211D at the position corresponding to position 211 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, the amino acid substitution N211Q at the position corresponding to position 211 of SEQ ID NO:1.

In further embodiments of the invention, the cutinase used in the agent according to the invention is a cutinase having at least 65% sequence identity with the amino acid sequence set forth in SEQ ID NO:1 over the entire length thereof and having, based on the numbering according to SEQ ID NO:1, at least one of the amino acid substitution pairs T61S and F208E, D63T and N211E, S66A and V177P, S66C and N211D, R89L and W155Y, R89K and W155A, F90I and N211Q, and Y92D and V177P at the positions corresponding to positions 61 and 208, 63 and 211, 66 and 177, 66 and 211, 89 and 155, 90 and 211, and 92 and 177 of SEQ ID NO:1.

In further embodiments of the invention, the invention also comprises cutinases derived from the amino acid sequences set forth in SEQ ID NO:1, for example by mutagenesis.

In various further embodiments, the agents according to the invention may also contain cutinases obtainable by expression of the nucleotide sequences according to SEQ ID NO: 2. In one aspect of the invention, the invention also comprises the nucleotide sequences set forth in SEQ ID NO:2 as well as nucleotide sequences which are related to these nucleotide sequences, having over the entire length thereof at least 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 98.8%, 99.0%, 99.2%, 99.4% or 99.6% sequence identity, with the proviso that the native sequence coding for the cutinase is excluded.

In various embodiments of the invention, the cutinase comprises an amino acid sequence which has a sequence identity of at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99,0%, 99,2%, 99,4% or 99,6% to the amino acid sequence set forth in SEQ ID NO:1 and which has at least one amino acid substitution, preferably at least one amino acid substitution which is selected from the group consisting of T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to positions 61, 63, 66, 89, 90, 92, 155, 177, 208 and 211 based on the numbering according to SEQ ID NO: 1.

In various further embodiments, the agent is characterized in that
(a) the cutinase can be obtained from a cutinase as defined as above as a starting molecule by single or multiple conservative amino acid substitution; and/or
(b) the cutinase can be obtained from a cutinase as defined as above as a starting molecule by fragmentation, deletion mutagenesis, insertion mutagenesis, or substitution mutagenesis and comprises an amino acid sequence that matches the starting molecule over a length of at least 169, 180, 190, 200, 210, 220, 230, 240, 245, 250, 251, 252, 253, 254, 255, 256, 257, 258, 259 or 260 interconnected amino acids, the at least one amino acid substitutions contained in the starting molecule still being present in the at least one position selected from the positions that correspond to positions 61, 63, 66, 89, 90, 92, 155, 177, 208 and 211 in SEQ ID NO:1.

The agents according to the invention preferably comprise the cutinase in an amount of 0.00001 to 1 wt.%, more preferably in an amount of 0.0001 to 0.5 wt.%, more particularly preferred in an amount of 0.001 to 0.1 wt.%, in each case based on the active protein.

In the context of the present invention, the feature that a cutinase has at least one of the specified amino acid substitutions means that it contains at the respective position one (or more) of the specified amino acid substitution(s), i.e. at least the specified positions are not otherwise mutated or deleted, e.g. by fragmentation of the cutinase.

The identity of nucleic acid or amino acid sequences is determined by a sequence comparison. Such a comparison is done by assigning similar sequences to one another in the nucleotide sequences or amino acid sequences. This sequence comparison is preferably performed on the basis of the conventionally used BLAST algorithm established in the prior art (cf. e.g. Altschul et al. (1990) "Basic local alignment search tool", J. Mol. Biol. 215:403-410, and Altschul et al. (1997) "Gapped BLAST and PSI-BLAST: a new generation of protein database search programs"; Nuc. Acids Res. 25:3389-3402) and in principle is done by assigning similar sequences of nucleotides or amino acids in the nucleic acid or amino acid sequences to one another. A tabular assignment of the positions in question is known as an alignment. A further algorithm available in the prior art is the FASTA algorithm. Sequence comparisons (alignments), in particular multiple sequence comparisons, are conventionally created using computer software. Use is often made for example of the Clustal series (cf. e.g. Chenna et al. (2003) "Multiple sequence alignment with the Clustal series of programs", Nuc. Acids Res. 31:3497-3500), T-Coffee (cf. e.g. Notredame et al. (2000) "T-Coffee: A novel method for multiple sequence alignments", J. Mol. Biol. 302:205-217) or programs which are based on these programs or algorithms. For the purposes of the present invention sequence comparisons and alignments are preferably performed with the computer program Vector NTI® Suite 10.3 (Invitrogen Corporation, 1600 Faraday Avenue, Carlsbad, California, USA) using the preset default parameters.

Such a comparison allows a statement to be made about the similarity of the compared sequences. It is conventionally stated in percent identity, i.e. the proportion of identical nucleotides or amino acid residues therein or in an alignment of mutually corresponding positions. The broader term "homology" also includes consideration of amino acid substitutions conserved in amino acid sequences, thus amino acids with similar characteristics, since they generally have similar activities or functions within the protein. The similarity of the compared sequences may therefore also be stated in percent homology or percent similarity. Statements regarding identity and/or homology may be made over entire polypeptides or genes or only over individual domains. Homologous or identical domains of various nucleic acid or amino acid sequences are therefore defined by matches in the sequences. They often have identical or similar functions. They may be small and comprise only a few nucleotides or amino acids. Often such small domains exercise functions which are essential for the overall activity of the protein. It may therefore be meaningful to relate sequence matches only to individual, optionally small domains. If not stated otherwise, however, statements regarding identity or homology in the present application relate to the entire length of the nucleic acid or amino acid sequence indicated in each case.

In the context of the present invention, the indication that an amino acid position corresponds to a numerically designated position in SEQ ID NO:1 means that the corresponding position is associated with the numerically designated position in SEQ ID NO:1 in an alignment as defined above.

A further aspect of the invention therefore relates to cutinases which are characterized in that they have at least one and increasingly preferred two, three or four identical antigenic determinants with a cutinase used in an agent according to the invention. Due to their immunological similarities, such cutinases are structurally similar to the cutinases used in the agents according to the invention in that it can be assumed that they also have a similar function.

Further cutinases which can be used in agents according to the invention may be cutinases further comprising additional amino acid modifications, in particular amino acid substitutions, insertions or deletions, compared to the cutinase set forth in SEQ ID NO:1 and having at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q at at least one of the positions corresponding to positions 61, 63, 66, 89, 90, 92, 155, 177, 208 and 211of SEQ ID NO:1. Such cutinases are, for example, further developed by targeted genetic modification, i.e. by mutagenesis, and optimized for specific applications or with regard to special properties (e.g. with regard to their catalytic activity, stability, etc.). Furthermore, nucleic acids encoding the cutinases used can be introduced into recombination approaches and thus used to generate completely new cutinases or other polypeptides.

Thereby, the aim is to introduce specific mutations such as substitutions, insertions or deletions into the known molecules in order, for example, to improve the decomposition performance of enzymes according to the invention. For this purpose, in particular the surface charges and/or the isoelectric point of the molecules and thus their interactions with the substrate can be modified. For example, the net charge of the enzymes can be modified in order to influence the substrate binding, especially for use in composite decomposition or waste water treatment. Alternatively or in addition, the stability of the cutinase can be further increased by one or more corresponding mutations, thereby improving its decomposition performance. Advantageous properties of individual mutations, e.g. individual substitutions, can complement each other. A cutinase that has already been optimized with regard to certain properties, for example with regard to its activity and/or its decomposition performance, can therefore be further developed within the scope of the present invention.

For the description of substitutions which relate to exactly one amino acid position (amino acid exchanges), the following convention is applied: first the naturally present amino acid is designated in the form of the internationally common single letter code, then the associated sequence position and finally the inserted amino acid. Several substitutions within the same polypeptide chain are separated by slashes. With regard to insertions, additional amino acids are specified after the sequence position. With regard to deletions, the missing amino acid is replaced by a symbol, for example an asterisk or a dash, or a Δ is indicated before the corresponding position. For example, N58Q describes the substitution of asparagine at position 58 by glutamine, N58NA describes the insertion of alanine after the amino acid asparagine at position 58, and N58* or ΔN58 describe the deletion of asparagine at position 58. This nomenclature is known to experts in the field of enzyme technology.

A further aspect of the invention therefore relates to an agent containing a cutinase characterized in that it is obtainable from a cutinase as described above as a starting molecule by single or multiple conservative amino acid substitution. The term "conservative amino acid substitution" means the replacement (substitution) of one amino acid residue with another amino acid residue, which replacement does not result in a change in polarity or charge at the position of the replaced amino acid, e.g. the replacement of a non-polar amino acid residue with another non-polar amino acid residue. Conservative amino acid substitutions in the invention include, for example: G=A=S, I=V=L=M, D=E, N=Q, K=R, Y=F, S=T, G=A=I=V=L=M=Y=F=W=P=S=T.

Alternatively or in addition, the cutinase used in an agent according to the invention is characterized in that it is obtainable from a cutinase as a starting molecule by fragmentation, deletion, insertion or substitution mutagenesis and comprises an amino acid sequence which has a length of at least 169, 180, 190, 200, 210, 220, 230, 240, 245, 250, 251, 252, 253, 254, 255, 256, 257, 258, 259 or 260 interconnected amino acids, said cutinase having at least one amino acid substitution at at least one of the positions corresponding to positions 61, 63, 66, 89, 90, 92, 155, 177, 208 and 211. In different embodiments, the cutinases thus obtainable, even after mutagenesis/substitution, still have the sequence identities defined herein of at least 65% with the sequence according to SEQ ID NO:1.

For example, it is possible to delete individual amino acids from the termini or loops of the enzyme without losing or reducing hydrolytic activity. Furthermore, such fragmentation, deletion, insertion or substitution mutagenesis can also reduce the allergenicity of enzymes and thus improve their overall usability. It is advantageous that the enzymes retain their hydrolytic activity even after mutagenesis, i.e. their hydrolytic activity corresponds at least to that of the starting enzyme, i.e. in a preferred embodiment the hydrolytic activity is at least 80%, preferably at least 90%, of the activity of the starting enzyme. Other substitutions may also have beneficial effects. Both single and several interconnected amino acids can be substituted by other amino acids.

The further amino acid positions are defined by an alignment of the amino acid sequence of a cutinase contained in the agents according to the invention with the amino acid sequence of the cutinase set forth in SEQ ID NO:1. Furthermore, the assignment of the positions depends on the mature protein. This assignment is in particular also to be applied if the amino acid sequence of a cutinase according to the invention comprises a higher number of amino acid residues than the cutinase according to SEQ ID NO:1. Starting from the positions mentioned in the amino acid sequence of the cutinase according to SEQ ID NO:1, the positions of change in a cutinase contained in the agents according to the invention are those which are assigned to these positions in an alignment.

Advantageous positions for sequence modifications, in particular substitutions, of the cutinase, which are preferably significant when transferred to homologous positions of the cutinases according to the invention and which confer advantageous functional properties on the cutinase, are therefore the positions which correspond in an alignment to the positions described herein, i.e. in the numbering based on the amino acid sequence set forth in SEQ ID NO:1. The following amino acid residues are present at the mentioned positions in the wild type molecule of the cutinase according to SEQ ID NO:1: T61, D63, S66, R89, F90, Y92, W155, V177, F208, and N211.

In various embodiments, the cutinase may have one or more further amino acids at the N- or C-terminus in addition to the sequence set forth in SEQ ID NO:1. In certain embodiments, such N-terminal peptides may be the naturally occurring signal peptides for cutinase or a single methionine residue.

A further confirmation of the correct assignment of the amino acids to be modified, i.e. in particular their functional correspondence, can be provided by comparative experiments, according to which the two positions assigned to each other on the basis of an alignment are modified in the same way in both cutinases compared with each other and it is observed whether the enzymatic activity or decomposition performance is modified in the same way in both. If, for example, an amino acid substitution in a certain position of the cutinase according to SEQ ID NO:1 is accompanied by a change in an enzymatic parameter, for example an increase in the K_{M} value (Michaelis constant), and if a corresponding change in the enzymatic parameter, for example also an increase in the K_{M} value, is observed in a cutinase variant contained in an agent according to the invention, the amino acid substitution of which was achieved by the same introduced amino acid, then this is to be seen as confirmation of the correct assignment.

In various further embodiments, the agent is characterized in that it contains one or more additional ingredients selected from the group consisting of further enzymes, enzyme stabilizers, emulsifiers, reducing agents, and antioxidants.

All percentages given in connection with the compositions/agents described herein refer, unless explicitly stated otherwise, to weight percent (wt.%) of the respective composition/agent.

Polyester treatment agents according to invention may contain further enzymes besides cutinase. These can be hydrolytic enzymes or other enzymes in a concentration appropriate for the effectiveness of the agent. An embodiment of the invention therefore represents agents comprising one or more enzymes. The preferred enzymes to be used are all enzymes which are able to develop catalytic activity in the invention, in particular a protease, amylase, cellulase, hemicellulase, mannanase, tannase, xylanase, xanthanase, xyloglucanase, β-glucosidase, pectinase, carrageenase, perhydrolase, oxidase, oxidoreductase, hydrolase, PETase or a lipase, as well as mixtures thereof. Enzymes are advantageously present in the agent in an amount of 1 x 10⁻⁸ to 5 wt.% based on active protein. Increasingly preferred, each enzyme is contained in the agents according to the invention in an amount of 1 x 10⁻⁷ to 3 wt.%, of 0.00001 to 1 wt.%, of 0.00005 to 0.5 wt.%, of 0.0001 to 0.1 wt.% and particularly preferred of 0.0001 to 0.05 wt.% based on active protein. The enzymes show particularly preferred synergistic performance with respect to decomposition of PET Synergistic effects can occur not only between different enzymes, but also between one or more enzymes and other ingredients of the agent according to the invention.

The protein concentration can be determined using known methods, for example the BCA method (bicinchonic acid; 2,2'-biquinolyl-4,4'-dicarboxylic acid) or the Biuret method. The active protein concentration is determined by titration of the active centers using a suitable irreversible inhibitor (e.g. phenyl methyl sulfonyl fluoride (PMSF) for proteases) and determination of the residual activity (see M. Bender et al., J. Am. Chem. Soc. 88, 24 (1966), p. 5890-5913).

In the agents described herein, the enzymes to be used may also be packaged together with accompanying substances, e.g. from fermentation. In liquid formulations, the enzymes are preferably used as liquid enzyme formulation(s).

The enzymes are usually not provided in the form of pure protein, but rather in the form of stabilized preparations that can be stored and transported. These preparations include, for example, the solid preparations obtained by granulation, extrusion or lyophilization or, in case of liquid or gel preparations in particular, enzyme solutions which are advantageously as concentrated as possible, low in water and/or mixed with stabilizers or other additives.

Alternatively, the enzymes can be encapsulated both for the solid and for the liquid dosage form, for example by spray-drying or extrusion of the enzyme solution together with a preferably natural polymer or in the form of capsules, for example those in which the enzymes are enclosed as in a solidified gel or in those of the core-shell type in which an enzyme-containing core is coated with a protective layer impermeable to water, air and/or chemicals. Additional active substances such as stabilizers, emulsifiers, pigments, bleaching agents or dyes can also be applied in deposited layers. Such capsules are applied according to methods known per se, for example by shaking or roll granulation or in fluid-bed processes. Advantageously, such granulates are low in dust, e.g. by applying polymer film formers, and are storage-stable due to the coating.

Furthermore, it is possible to assemble two or more enzymes together so that a single granulate has several enzyme activities.

In various embodiments, the invention may contain one or more enzyme stabilizers. Therefore, the invention may also contain an enzyme stabilizer selected from the group consisting of sodium formate, sodium sulfate, lower aliphatic alcohols and boric acid and their esters and salts. Of course, two or more of these compounds can also be used in combination. The salts of these compounds can also be used in the form of hydrates, such as sodium sulfate decahydrate.

The term "lower aliphatic alcohols" as used herein includes mono-alcohols, diols and higher alcohols with up to 6 carbon atoms. In this context, polyols such as glycerol, (mono)ethylene glycol, (mono)propylene glycol or sorbitol are particularly mentioned as belonging to the group of lower aliphatic alcohols, without the invention being limited to them.

In addition to at least one enzyme stabilizer selected from the above group, an agent according to the invention may also contain at least one further stabilizer. Such stabilizers are known in the state of the art.

Reversible protease inhibitors protect the enzymes contained in a treatment agent from proteolytic degradation by reversibly inhibiting the enzymatic activity of the proteases contained in the agent. Benzamidine hydrochloride, boronic acids or their salts or esters are frequently used as reversible protease inhibitors, among them mainly derivatives with aromatic groups, such as ortho-, meta- or para-substituted phenyl boronic acids, in particular 4-formyl phenyl boronic acid, or the salts or esters of the compounds mentioned. Peptide aldehydes, i.e. oligopeptides with reduced C-terminus, in particular those consisting of 2 to 50 monomers, are also used for this purpose. Peptide reversible protease inhibitors include ovomucoid and leupeptin.

Further enzyme stabilizers are amino alcohols such as mono-, di-, triethanol- and propanolamine and mixtures thereof, aliphatic carboxylic acids up to C12, such as succinic acid, other dicarboxylic acids or salts of these acids. End-capped fatty acid amide alkoxylates are also suitable for this purpose. Some organic acids used as builders are also able to act as enzyme stabilizers. Calcium and/or magnesium salts, such as calcium acetate, are also used for this purpose.

Polyamide oligomers or polymeric compounds such as lignin, water-soluble vinyl copolymers or cellulose ethers, acrylic polymers and/or polyamides stabilize the enzyme preparation against physical influences or pH fluctuations, among other things. Polymers containing polyamine-N-oxide act simultaneously as enzyme stabilizers and color transfer inhibitors. Other polymeric stabilizers are linear C8-C18 polyoxyalkylenes. Alkyl polyglycosides can also stabilize the enzymatic components of the agent according to the invention and are preferably able to additionally increase their performance. Crosslinked N-containing compounds preferably fulfil a dual function as soil-release agents and as enzyme stabilizers. Hydrophobic, non-ionic polymer stabilizes in particular a cellulase which may be present.

Reducing agents and antioxidants increase the stability of enzymes against oxidative degradation; for this purpose, sulphur containing reducing agents such as sodium sulfite and reducing sugars are common.

In one embodiment, the agents according to the present invention are liquid and contain water as main solvent, i.e. they are aqueous agents. The water content of the aqueous agent according to the invention is usually 1 to 70 wt.%, preferably 3 to 65 wt.%, more preferred 5 to 60 wt.-%. In various embodiments, the water content is more than 5 wt.%, preferably more than 15 wt.% and particularly preferred more than 50 wt.%, based in each case on the total amount of agent.

In addition, non-aqueous solvents can be added to the agent. Suitable non-aqueous solvents include monohydric or polyhydric alcohols, alkanolamines or glycol ethers, provided they are miscible with water within the specified concentration range. The solvents shall preferably be selected from ethanol, n-propanol, i-propanol, butanols, glycol, propanediol, butanediol, methyl propanediol, glycerol, diglycol, propyl diglycol, butyl diglycol, hexylene glycol, ethylene glycol methyl ether, ethylene glycol ethyl ether, ethylene glycol propyl ether, ethylene glycol mono-n-butyl ether, diethylene glycol methyl ether, diethylene glycol ethyl ether, propylene glycol methyl ether, propylene glycol ethyl ether, propylene glycol propyl ether, dipropylene glycol monomethyl ether, dipropylene glycol monomethyl ether, methoxytriglycol, ethoxytriglycol, butoxytriglycol, 1-butoxyethoxy-2-propanol, 3-methyl-3-methoxybutanol, propylene glycol t-butyl ether, di-n-octyl ether and mixtures of these solvents.

The one or more non-aqueous solvents is/are usually contained in an amount of 0.1 to 25 wt.%, preferably 0,5 to 10 wt.%, more preferred 1 to 8 wt.%, based on the total amount of agent.

In various further embodiments, the agent is characterized in that the cutinase is immobilized.

In the context of the present invention, "immobilized" is understood to mean that the cutinase is linked to a solid carrier material. The solid carrier material may preferably be present in particulate form.

In a further embodiment, the cutinase is immobilized by chemically or physically bonding the cutinase to a carrier material, preferably a solid carrier. Physical linking may be achieved by adsorption of the cutinase to the carrier material. Chemical linking may be achieved by chemically binding the cutinase to the surface of the carrier material. Preferably, chemical linking is achieved by binding functional groups of amino acid residues of the cutinase to chemical surface functionalities of the solid carrier material. The chemical surface functionalities and functional groups of the amino acid residues of the cutinase are preferably selected from the group consisting of-SH, -OH, -COOH, -NH₂, or -CH₂Cl.

In a further embodiment, the solid carrier material is a porous material. Preferably the material is selected from the group consisting of organic or inorganic polymers such as for example silica gel or polystyrene, and/or inorganic or inorganic/organic hybrid materials such as magnetic particles, zeolites, and/or metal organic frameworks.In a further embodiment, the solid carrier is at least one solid carrier selected from the group consisting of Merrifield resin, polyacrylamide resin, TentaGel resin, Wang resin, safety-catch linker, traceless linker, photolabile linker, or controlled pore glass (CPG).

In a further embodiment, the carrier is at least one carrier selected from the group consisting of non-cross-linked polystyrene, polyvinyl alcohol, or polyethylene glycol.

A further aspect of the invention relates to a method for treatment of a polyester composition, in particular a PET composition, characterized in that in at least one method step an agent according to the invention is added to the polyester composition, wherein the cutinase is reacted with the polyester.

In various embodiments, the method described above is characterized by that the invention is used at a temperature of 0-100°C, preferably 0-80°C, further preferably 20-60°C and most preferred 20-40°C.

In various embodiments, the method according to the invention is characterized in that the cutinase at least partially decomposes the polyester into oligomers and/or monomers, particularly into biodegradable oligomers, dimers and/or monomers.

Particularly, if the polyester is PET, the cutinase decomposes the polyester into oligomers and/or dimers of PET and/or the monomers terephthalic acid and ethylene glycol.

In various embodiments, the method according to the invention is characterized in that the polyester composition is water comprising polyester particles, in particular micro particles, or in that the polyester composition is an aqueous suspension of composite materials comprising polyester.

In various embodiments, the method according to the invention is characterized in that the cutinase is deactivated and/or decomposed after reacting with the polyester, particularly by adding an enzyme inhibitor, an enzyme decomposing the cutinase, a strong acid, or a strong base to the polyester composition or by heating the polyester composition, particularly above 80°C, after reacting the cutinase with the polyester.

In various embodiments, the method according to the invention is characterized in that the cutinase is immobilized and in that the immobilized cutinase is removed after reacting with the polyester by physical and/or mechanical means, in particular by filtration, centrifugation, sedimentation, flotation, extraction and/or magnetic separation.

Thereby, it is possible to retrieve the cutinase after performing the method according to the present invention in order to recycle the cutinase. The retrieved cutinase may be used again in an agent for the same or a different process. Further washing and recycling steps may be performed after retrieving the cutinase from the reaction.

In waste water or sewage treatment applications the agent according to the invention can be used to degrade microplastic particles consisting of PET like polymer filaments, fibres or other kinds of PET-based product debris and fragments (particles). The efficiency of the degradation process depends on the nature and size of the PET-particles. The smaller the particles, the quicker the degradation process works. The enzymes in the agent are water soluble and can be used in basically any kind of reactor suitable for handling of liquid reactants and under versatile conditions. If necessary, the enzyme in the agent can be further adapted to the required conditions by enzyme engineering techniques, e.g. stability against temperature, pH, solvents and other properties can be adapted (Scientific literature on lipases and esterases).

The reaction products stemming from the degradation are terephatalic acid and ethylene glycol as well as the agent itself are biodegradable and can be either degraded by further waste water treatment or otherwise taken care of.

Should it be necessary to inactivate the enzyme in the agent after completion of the process, this can be achieved either by heating it up to an appropriate temperature. Usually enzymes are deactivated by temperatures of above 80°C and denature in boiling water.

The enzymes in the agent can also be deactivated by addition of enzyme inhibitors, or by applying extreme pH-values.

The enzymes in the agent can also be degraded by other enzymes like proteases. Such proteases can be immobilized (bound) on a solid carrier (particles) which can be filled into a cartridge or pipe through which a liquid can be pumped. Proteins in such liquid that make contact to the proteases on the carrier particles would then be degraded and hence deactivated. The carrier particles are held back by a filter membrane or sieve so that they remain inside the cartridge while the cleaned liquid or composition can flow out of the cartridge and can be subjected to further processing or other use.

In a further aspect, the invention relates to the use of a cutinase in waste water, sewage, or composite material solution treatment, wherein the cutinase has at least 65% sequence identity with the amino acid sequence set forth in SEQ ID NO:1 over the entire length thereof and has, with respect to the numbering according to SEQ ID NO:1, at least one amino acid substitution, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to the positions 61, 63, 66, 89, 90, 92, 155, 177, 208 and 211.

A further aspect of the invention relates to a method for the production of a cutinase used in the agent and method according to the invention, wherein the cutinase is expressed by a system selected from the group consisting of *E. coli, Bacillus subtilis, and Bacillus lichniformis.*

All facts, objects and embodiments described for agents according to the invention and the cutinase are also applicable to the other aspects of the invention. Therefore, reference is expressly made here to the disclosure at the appropriate place with the note that this disclosure also applies to the above methods according to the invention and the uses according to the invention.

### Examples

### Example 1: Cloning and Expression

### E. coli BL21 (pLyS) and BL21 DE3

Vector (6,162 bp): pET_22b
Subcloning sites: NdeI/XhoI (802 bp)

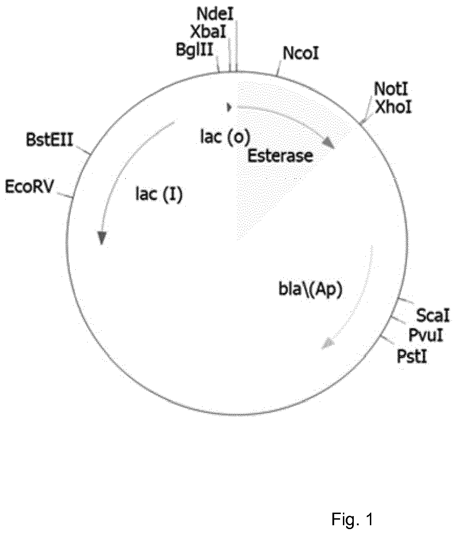

As shown in Fig. 1 the nucleotide sequence encoding for a protein having cutinase-like esterase activity was synthesized and subcloned at the restriction sites NdeI and XhoI in the pET 22 b vector backbone resulting in the vector pET_22b-Esterase 3-13. The sequence was also subcloned including a his-tag at the C-terminal. The sequence was codon optimized for its expression in *E. coli.* The original sequence was previously isolated from a gene library that was constructed using a metagenome derived from a foliage compost. (WO2012099018 (A1)). The vector was transformed in *E. coli* BL21 DE3. The sequence of the plasmid was confirmed by restriction pattern and sequencing.

### E. coli expression

Culture conditions in shake flask using *E. coli* BL21 DE3 transformed with pET_22b-Esterase 3-13:
The strain was cultured in 3 ml of LB medium (1% (w/v) Bacto tryptone, 0.5% (w/v) yeast extract and 1% (w/v) NaCl) with ampicillin (100 µg/ml) at 37°C and 180rpm for 12 hours. One ml of this culture was used to inoculate 100 ml of LB ampicilin in 500 ml shake flasks. Once the culture reaches a OD of 0.9 at 600 nm, the expression was induced using 0.5 mM IPTG carried out at OD (600 nm) of 0.9. The temperature was reduced to 30°C and the culture was induced for 4 hours.

The activity of the cutinase-like esterase is determined spectrophotometrically by the hydrolysis of p-NPC. The increased absorbance at 410 nm is monitored for 5 minutes at 37°C. Change of absorption must be linear over the period of measurement.

### Production of the Variants in E. coli:

The cutinase-like esterase variants, both expressed in *E. coli* (*E. coli* BL21 DE3) are produced at 5L bioreactor operated at 3 L working volume. After 48 h fed-batch fermentation, the cells were harvested and the supernatant fraction and the pellet fraction (biomass) were separated by centrifugation.

### Downstream processing:

The biomass was disrupted using ultrasound and further centrifugation (20 min, 8,000 rpm, 4°C). The supernatant was heated at 70°C for 10 minutes and centrifuged at 15,000 rpm, 4°C for 20 min to remove the insoluble fraction.

### Example 2: Waste water treatment by free floating enzymes

A process solution (waste water or sewage) or composition of PET-particle-containing material is filled into a vessel like a reactor, a tank, basin or alike. The agent according to the invention is added to the PET-containing composition and mixed. Addition can be performed by adding the enzyme before filling of the tank or basin by mixing the enzyme into the feedstream (inlet). The agent exerts its activity right away in that it binds to the PET particles and start degrading them depending on their nature, size, accessibility, reaction conditions like temperature, pH, presence of solvents or other interfering agents etc. The solution is allowed to react until the appropriate level of degradation is achieved.

Degradation can be analyzed by standard methodology like HPLC or by other analytical methods depending on the composition of the PET-containing material/liquid and the nature of the PET-particles.

To inactivate the inventive agent after completion of the process, the process solution is heated up to an appropriate temperature, e.g. 85°C.

Alternatively, the enzyme in the inventive agent is degraded by other enzymes, like proteases. Such proteases are immobilized (bound) on a solid carrier (particles) which is filled into a cartridge or pipe through which a process solution after degradation of the PET-particles is pumped. Proteins in the process solution make contact to the proteases on the carrier particles will be degraded and hence deactivated. The carrier particles are held back by a filter membrane or sieve so that they remain inside the cartridge while the cleaned process solution or composition flows through the cartridge.

In waste water plants the inventive agent can be used at any stage of purification process, while use as a final process step after general purification of the waste water or sewage is preferred to optimize the efficiency of the PET-degradation process by the inventive agent.

After inactivation of the inventive agent the process solution is dispatched to the sewage system.

### Example 3: Waste water treatment by immobilized enzymes

A process solution (waste water or sewage) or composition of PET-particle-containing material is guided through a cartridge or pipe filled with a comprises comprising a cutinase, which has at least 65% sequence identity with the amino acid sequence set forth in SEQ ID NO:1 over the entire length thereof and has, with respect to the numbering according to SEQ ID NO:1, at least one amino acid substitution, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to the positions 61, 63, 66, 89, 90, 92, 155, 177, 208 and 211. In the composition the cutinase is immobilized by binding the enzyme to a solid carrier material or a porous carrier material. Preferably, the carrier material is selected from the group consisting of organic or inorganic polymers such as for example silica gel or polystyrene, and/or inorganic or inorganic/organic hybrid materials such as magnetic particles, zeolites, and/or metal organic frameworks. The immobilized enzyme exerts its activity right away in that it binds to the PET particles and start degrading them depending on their nature, size, accessibility, reaction conditions like temperature, pH, presence of solvents or other interfering agents etc. The solution is allowed to react with the immobilized enzyme until the appropriate level of degradation is achieved. To allow sufficient degradation the flow rate of the solution through the cartridge or pipe can be varied.

Degradation can be analyzed by standard methodology like HPLC or by other analytical methods depending on the composition of the PET-containing material/liquid and the nature of the PET-particles.

In waste water plants the inventive agent can be used at any stage of purification process, while use as a final process step after general purification of the waste water or sewage is preferred to optimize the efficiency of the PET-degradation process by the inventive agent.

After inactivation of the inventive agent the process solution is dispatched to the sewage system.

### Example 4: Composite material degradation

A composite material comprising PET is comminuted by appropriate means, like a shredder, comminutor, macerator, grinder or the like. The comminuted material is transformed into an aqueous slurry by addition of water and, optionally, additives like surfactants or detergents. The aqueous slurry is filled into a reactor tank. The agent according to the invention is added to the slurry and mixed by stirring or alternative mixing actions. The agent exerts its activity right away in that it binds to the PET particles of the composite material and start degrading them depending on their nature, size, accessibility, reaction conditions like temperature, pH, presence of solvents or other interfering agents etc. The solution is allowed to react until the appropriate level of degradation is achieved.

Degradation can be analyzed by standard methodology like HPLC or by other analytical methods depending on the composition of the PET-containing material/liquid and the nature of the PET-particles.

To inactivate the inventive agent after completion of the process, the process solution is heated up to an appropriate temperature, e.g. 85°C.

Alternatively, the enzyme in the inventive agent is degraded by other enzymes, like proteases. Such proteases are immobilized (bound) on a solid carrier (particles) which is filled into a cartridge or pipe through which a process solution after degradation of the PET-particles is pumped. Proteins in the process solution make contact to the proteases on the carrier particles will be degraded and hence deactivated. The carrier particles are held back by a filter membrane or sieve so that they remain inside the cartridge while the cleaned process solution or composition flows through the cartridge.

Preferably, prior to the inactivation of the enzyme, the slurry is subject to a separation or filtration step by which the remaining particular components of the composite material, like e.g. paper or metal foil, is separated from the aqueous solution.

## Claims

1. Polyester compositions treatment agent, in particular PET compositions treatment agent, **characterized in that** the agent comprises a cutinase, which has at least 65% sequence identity with the amino acid sequence set forth in SEQ ID NO:1 over the entire length thereof and has, with respect to the numbering according to SEQ ID NO:1, at least one amino acid substitution, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to the positions 61, 63, 66, 89, 90, 92, 155, 177, 208 and 211.

2. Agent according to claim 1, **characterized in that** the cutinase comprises an amino acid sequence which has at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,8%, 99,0%, 99,2%, 99,4% or 99,6% sequence identity with the amino acid sequence set forth in SEQ ID NO:1 over the entire length thereof and has, with respect to the numbering according to SEQ ID NO:1, at least one amino acid substitution, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to the positions 61, 63, 66, 89, 90, 92, 155, 177, 208 and 211.

3. Agent according to claim 1 or 2, **characterized in that**
the cutinase can be obtained from a cutinase as defined as above as a starting molecule by single or multiple conservative amino acid substitution; and/or
the cutinase can be obtained from a cutinase as defined as above as a starting molecule by fragmentation, deletion mutagenesis, insertion mutagenesis, or substitution mutagenesis and comprises an amino acid sequence that matches the starting molecule over a length of at least 169, 180, 190, 200, 210, 220, 230, 240, 245, 250, 251, 252, 253, 254, 255, 256, 257, 258, 259 or 260 interconnected amino acids, the at least one amino acid substitutions contained in the starting molecule still being present in the at least one position selected from the positions that correspond to positions 61, 63, 66, 89, 90, 92, 155, 177, 208 and 211 in SEQ IDNO:1.

4. Agent according to any one of claims 1 to 3, wherein the cutinase is contained in the agent in an amount from 0,00001 to 1 wt.%, preferably in an amount from 0,0001 to 0,5 wt.%, particularly preferred in an amount from 0,001 to 0,1 wt.%.

5. Agent according to any one of claims 1 to 4, **characterized in that** it contains one or more additional ingredients selected from the group consisting of further enzymes, stabilizers, emulsifiers, reducing agents, and antioxidants.

6. Agent according to any one of claims 1 to 5, **characterized in that** the cutinase is immobilized.

7. Agent according to any one of the claims 1 to 6, **characterized in that** the cutinase is expressed by a system selected from the group consisting of *E. coli, Bacillus subtilis, and Bacillus lichniformis.*

8. Method for treatment of a polyester composition, in particular a PET composition, **characterized in that** in at least one method step an agent according to any one of claims 1 to 7 is added to the polyester composition, wherein the cutinase is reacted with the polyester.

9. Method according to claim 8, **characterized in that** the polyester composition is an aqueous suspension of particles, wherein the particles comprise polyester.

10. Method according to any one of claims 8 or 9, **characterized in that** the cutinase at least partially decomposes the polyester into oligomers, dimers and/or monomers, particularly into biodegradable oligomers and/or monomers.

11. Method according to any one of claims 8 to 10, **characterized in that** the polyester composition is water comprising polyester particles, in particular micro particles, or **in that** the polyester composition is an aqueous suspension of composite materials comprising polyester.

12. Method according to any one of claims 8 to 11, **characterized in that** the cutinase is deactivated or decomposed after reacting with the polyester, particularly by adding an enzyme inhibitor, an enzyme decomposing the cutinase, a strong acid, or a strong base to the polyester composition or by heating the polyester composition, particularly above 80°C, after reacting the cutinase with the polyester.

13. Method according to any one of claims 8 to 12, **characterized in that** the cutinase is immobilized and **in that** the immobilized cutinase is removed after reacting with the polyester by physical and/or mechanical means, in particular by filtration, centrifugation, sedimentation, flotation, extraction and/or magnetic separation.

14. Use of a cutinase in waste water, sewage, or composite material solution treatment, wherein the cutinase has at least 65% sequence identity with the amino acid sequence set forth in SEQ ID NO:1 over the entire length thereof and has, with respect to the numbering according to SEQ ID NO:1, at least one amino acid substitution, in particular at least one of the amino acid substitutions T61S, D63E, D63T, S66A, S66C, R89L, R89K, F90I, Y92D, Y92C, Y92A, W155A, W155Y, V177P, F208E, F208Q, F208Y, N211E, N211D, and N211Q, at at least one of the positions corresponding to the positions 61, 63, 66, 89, 90, 92, 155, 177, 208 and 211.
